Europäisches Patentamt

European Patent Office

Office européen des brevets

(19)

(11) **EP 0 928 786 B1**

(12) **EUROPEAN PATENT SPECIFICATION**

(45) Date of publication and mention
of the grant of the patent:
**02.01.2003 Bulletin 2003/01**

(51) Int Cl.⁷: **C07C 311/19**, A61K 38/55,
C07K 5/06, C07D 213/71

(21) Application number: **99102501.6**

(22) Date of filing: **23.10.1996**

(54) **Angiogenesis inhibitor**

Angiogenese Inhibitoren

Inhibiteurs da la angiogenesis

(84) Designated Contracting States:
**AT BE CH DE DK ES FI FR GB GR IE IT LI LU MC
NL PT SE**

(30) Priority: **25.10.1995 JP 27748595
19.09.1996 JP 24804696**

(43) Date of publication of application:
**14.07.1999 Bulletin 1999/28**

(60) Divisional application:
**99106760.4 / 0 927 716**

(62) Document number(s) of the earlier application(s) in
accordance with Art. 76 EPC:
**96116994.3 / 0 771 565**

(73) Proprietor: **Senju Pharmaceutical Co., Ltd.
Osaka-shi, Osaka 541-0046 (JP)**

(72) Inventors:
• **Fukiage, Chiho
Katano-shi, Osaka 576 (JP)**
• **Azuma, Mitsuyoshi
Nishinomiya-shi, Hyogo 662 (JP)**
• **Inoue, Jun 603, Rui-Shatore Suma Myodani
Kobe-shi, Hyogo 654-01 (JP)**
• **Nakamura, Masayuki
Kita-ku, Kobe-shi, Hyogo 651-1142 (JP)**
• **Yoshida, Yuka
Nishiwaki-shi, Hyogo 667 (JP)**

(74) Representative:
**von Kreisler, Alek, Dipl.-Chem. et al
Patentanwälte,
von Kreisler-Selting-Werner,
Bahnhofsvorplatz 1 (Deichmannhaus)
50667 Köln (DE)**

(56) References cited:
**EP-A- 0 393 457      EP-A- 0 504 938
EP-A- 0 611 756      EP-A- 0 623 627
EP-A- 0 731 107      WO-A-96/10014**

• **P BAGAVANDOSS ET AL: "Recombinant
Truncated Thrombospondin-1 monomer
Modulates Endothelial Cell Plasminogen
Activator Inhibitor-1 Accumulation and
Proliferation in vitro" BIOCHEMICAL AND
BIOPHYSICAL RESEARCH COMMUNICATIONS,
vol. 2, no. 192, 1 January 1993, page 325 32
XP002076315**

Remarks:
The file contains technical information submitted
after the application was filed and not included in this
specification

**Description**

Technical Field

The present invention relates to an angiogenesis inhibitor comprising a cysteine protease inhibitory compound.

Background of the Invention

An angiogenesis is a phenomenon wherein new blood vessels are created to form a new vascular network in the living body. The angiogenesis is found under normal physiological environment such as genesis and reproduction with regard to embryo, fetus, placenta, uterus and the like. It is also a pathologic phenomenon which accompanies wound healing, inflammation, growth of tumor and the like, and which is ophthalmologically seen in diabetic retinopathy, prematurity retinopathy, retinal venous occlusion, senile discoid macular degeneration and the like.

The angiogenesis greatly varies depending on the function and growth of endothelial cells, and is considered to be a cascade reaction which proceeds in the smallest vein along the following steps. That is, new blood vessels are presumably formed as a result of consecutive elementary reactions of (1) activation of vascular endothelial cells which are in the stage of rest upon differentiation, (2) destruction of cell matrix such as basement membrane by endothelial cells which expressed protease activity, (3) migration of endothelial cells, (4) proliferation of endothelial cells and (5) tube-formation by differentiation of endothelial cells [T. Oikawa, Drug News Perspest, Vol. 6, pp. 157-162 (1993)]. Each step of these reactions has been clarified to be promoted by angiogenesis promoters. Such angiogenesis promoters include, for example, blood vessel inducing factors [e.g., tumor angiogenetic factor (TAF)] secreted from tumor tissues, and growth factors such as fibroblast growth factor (FGF) present in various normal tissues, endothelial cell growth facor derived from platelets and vascular endothelial cell growth factor. In addition, cytokine, prostaglandine, monobutylin and angiogenine reportedly have similar direct or indirect effects [M. Klagsbrun et al., Annu. Rev. Physiol., Vol. 53, pp. 217-239 (1991)].

A substance which suppresses such angiogenesis include angiostatic steroids [Folkman, J. et al., Science, Vol. 221, p. 719 (1983)] such as cortisone which inhibits growth of endothelial cells; medroxyprogesterone acetate which inhibits production of plasminogen activator by endothelial cells; fumagillin acid derivatives which inhibit proliferation of endothelial cells and tube-formation; polysaccharide sulfate SD-4152 which inhibits proliferation and migration of endothelial cells; and retinoic acid which is responsible for modification of endothelial cell differentiation [Tsutomu Oikawa, *Kekkan to Naihi*, vol. 2, pp. 470-480 (1992)].

However, the above-mentioned drugs which inhibit angiogenesis have not been complete therapeutic agents for clinically suppressing angiogenesis, since some of them cause strong side-effects, thereby posing problems in terms of safety, and others only show insufficient effects.

EP-A-0 611 756 discloses alcohol or aldehyde derivatives as cathepsin L inhibitor and bone resorption inhibitors.

EP-A-0 504 938 discloses prophylactic and therapeutic compositions for bone diseases comprising di- or tripeptide derivatives as active ingredients.

EP-A-0 623 627 describes peptide sulfonamide derivatives showing inhibitor activity against cysteine protease.

EP-A-0 393 457 describes N-substituted peptidyl aldehydes and their use as cysteine proteinase inhibitors.

EP-A-0 731 107 discloses the production of aldehyde derivatives useful as bone resorption inhibitors.

WO 96/10014 describes acylaminoaldehyde derivatives showing inhibitory action against cysteine protease.

Summary of the Invention

It is therefore an object of the present invention to provide a pharmaceutical agent which provides strong angiogenesis-inhibitory effects.

According to the present invention, there has been provided an angiogenesis inhibitor comprising a cysteine protease inhibitory compound.

Cysteine protease is a protease having a cysteine residue in the active site of the enzyme molecule and includes such species as cathepsin B, H, and L and dipeptidyl peptidase, all of which are lysosomal enzyme fractions, and calpain which occurs in the cytoplasm, among others. Though much remains to be explored about the physiological roles of these enzymes, a considerable amount of light has been cast on their roles in recent years. For example, calpain is known to be a protease ubiquitous in life, which is activated by calcium ions and has the optimum pH in neutral. As elucidated to this day, it takes part in degradation of the skeletal protein of cells, activation of inert cell precursors such as protein kinase C, and degradation of receptor proteins. It has also been shown that the abnormality of this enzyme activity is involved in many diseases. For example, its involvement in refractory diseases such as cerebral apoplexy (stroke), subarachnoid hemorrhage, Alzheimer's disease, ischemic diseases, muscular dystrophy, cataract, platelet aggregation disorder, arthritis, and osteoporosis, among other diseases. [Trends in Pharmacological Science, Vol. 15, p. 412 (1994)].

The present invention relates to

(1) N-(4-Fluorophenylsulfonyl)-L-valyl-L-leucinal or a salt thereof;
(2) a pharmaceutical composition comprising N-(4-Fluorophenylsulfonyl)-L-valyl-L-leucinal or a salt thereof; and
(3) the use of N-(4-Fluorophenylsulfonyl)-L-valyl-L-leucinal or a salt thereof as an angiogenesis inhibitor.

The salts of N-(4-Fluorophenylsulfonyl)-L-valyl-L-leucinal are preferably physiologically acceptable ones, such as salts with inorganic base, salts with organic base, salts with inorganic acid, salts with organic acid, and salts with basic or acidic amino acid. Examples of preferable salts with inorganic base include alkali metal salts such as sodium salt and potassium salt; alkaline earth metal salts such as calcium salt and magnesium salt; and aluminum salt and ammonium salt. Examples of preferable salts with organic base include salts with trimethylamine, pyridine, picoline, ethanolamine, diethanolamine, triethanlolamine, dicyclohexylamine, and N,N-dibenzylethylenediamine.

Examples of preferable salts with inorganic acid include salts with hydrochloric acid, hydrobromic acid, nitric acid, sulfuric acid, and phosphoric acid. Examples of preferable salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Examples of preferable salts with basic amino acid include salts with arginine, lysine, and ornithine, and examples of preferable salts with acidic amino acid include salts with aspartic acid, and glutamic acid.

Cysteine protease inhibitory compounds can be administered systemically or locally. For systemic administration, they are administered orally or parenterally by, for example, intravenous injection, subcutaneous injection, intramuscular injection and the like. For local administration, they are administered transdermally, transmucosally, intranasally, intraocularly or other route.

Cysteine protease inhibitory compounds can be used to formulate pharmaceutical compositions. Examples of compositions to be orally administered to human include powders, granules, tablets, capsules, syrups and liquids. When the composition is prepared into powders, granules, tablets and the like, optional pharmaceutical carriers suitable for preparing solid compositions, such as vehicles (e.g., starch, glucose, fruit sugar and sucrose), lubricants (e.g., magnesium stearate), disintegrators (e.g., starch and crystalline cellulose), and binders (e.g., starch and gum arabic). The compositions may be coated with gelatin, sucrose and the like are admixed as appropriate. When the composition is syrup or liquid, for example, stabilizers (e.g., sodium edetate), suspending agents (e.g., gum arabic and carmellose), corrigents (e.g., simple syrup and glucose), aromatics may be used as appropriate. A parenteral composition may be injections or suppositories. When the composition is an injection, for example, solvents (e.g., distilled water for injection), stabilizers (e.g., sodium edetate), isotonizing agents (e.g., sodium chloride, glycerine and mannitol), pH adjusting agents (e.g., hydrochloric acid, citric acid and sodium hydroxide), suspending agents (e.g., methyl cellulose) may be used. When the composition is suppositories, for example, a base for suppositories such as cacao butter and macrogols, may be used as appropriate. Examples of compositions for external use include ointment, cream, lotion, collunarium and eye drop. These compositions for external use may contain, in addition to said inhibitory compound, for example, known compounds such as ointment base (e.g., petrolatum and lanolin), solvent (e.g., physiological saline and purified water), stabilizer (e.g., sodium edetate and citric acid), wetting agent (e.g., glycerine), emulsifier (e.g., polyvinylpyrrolidone), suspending agent (e.g., hydroxypropylmethylcellulose and methylcellulose), surfactant (e.g., polysorbate 80 and polyoxyethylene hydrogenated castor oil), preservative (e.g., benzalkonium chloride, p-hydroxybenzoate and chlorobutanol), buffer (e.g., boric acid, sodium tetraborate, sodium acetate, citrate buffer and phosphate buffer), isotonizing agent (e.g., sodium chloride, glycerol and mannitol), pH adjusting agent (e.g., hydrochloric acid and sodium hydrochloride) as appropriate.

The angiogenesis inhibitor of the present invention may contain other pharmaceutical ingredients such as antiinflammatory drug, antitumor drug and antimicrobial agent, and the like.

While the dose of the cysteine protease inhibitory compound may vary depending on target disease, symptom, administration target, administration route and the like, the amount per dose is generally 1-500 mg, preferably 10-200 mg by oral administration, and generally 0.1-100 mg, preferably 1-50 mg by injection. When the composition is administered locally, for example, an eye drop adjusted generally to 0.001-1.0 w/v%, preferably 0.01-0.5 w/v%, is instilled to the eye by 20-50 µl at a time for 5 or 6 times a day.

The present invention is described in more detail by way of Examples and Experimental Examples in the following, which by no way limit the present invention. The following Reference Examples, Examples and Experimental Examples are all intended to describe the present invention in further detail and should by no means be construed as defining the scope of the invention.

Example 1

N-(2-Naphthalenesulfonyl)-L-valyl-L-leucinal

Valine (11.5 g) was dissolved in 1M aqueous sodium hydroxide solution (100 ml), and purified water (200 ml) and tetrahydrofuran (100 ml) were added. Thereto were simultaneously added dropwise 1M aqueous sodium hydroxide solution (100 ml) and a solutiion (100 ml) of 2-naphthalenesulfonyl chloride (18.5 g) in tetrahydrofuran with stirring under ice-cooling. The solution was stirred for one day at room temperature to allow reaction. After the completion of the reaction, the reaction mixture was adjusted to pH 2-3 and extracted with ethyl acetate. The extract was washed with dilute hydrochloric acid and saturated brine, and dried over anhydrous magnesium sulfate. Ethyl acetate was evaporated under reduced pressure, and the residue was washed with a mixture of hexane-ethyl acetate to give 12.8 g of N-(2-naphthalenesulfonyl)-L-valine as white crystals.

N-(2-Naphthalenesulfonyl)-L-valine (12. 0 g) and N-hydroxysuccinimide (5.4 g) were dissolved in tetrahydrofuran (200 ml), and a solution (200 ml) of 1-ethyl-3-(3-dimethylaminopropyl)carbodiimide hydrochloride (9.0 g) in dichloromethane was gradually added dropwise with stirring under ice-cooling. The solution was stirred for 4 hr at room temperature to allow reaction. After the completion of the reaction, the solvent was evaporated under reduced pressure and the residue was dissolved in ethyl acetate. The mixture was washed with dilute hydrochloric acid, saturated aqueous solution of sodium hydrogencarbonate and saturated brine, and dried over anhydrous magnesium sulfate. Ethyl acetate was evaporated under reduced pressure, and the residue was washed with a mixed solution of hexane-ethyl acetate to give 14.1 g of N-(2-naphthalenesulfonyl)-L-valine N-hydroxysuccinimide ester as white crystals.

N-(2-Naphthalenesulfonyl)-L-valine N-hydroxysuccinimide ester (1.8 g) and leucinol (0.63 g) were added to dichloromethane (100 ml), and the mixture was stirred at room temperature while adding triethylamine (0.68 g). The solution was stirred for 2 hr to allow reaction. After the completion of the reaction, the mixture was washed with dilute hydrochloric acid, saturated aqueous solution of sodium hydrogencarbonate and saturated brine, and dried over anhydrous magnesium sulfate. Dichloromethane was evaporated under reduced pressure, and the residue was washed with a mixed solution of hexane-ethyl acetate to give 1.3 g of N-(2-naphthalenesulfonyl)-L-valyl-L-leucinol as white crystals.

N-(2-Naphthalenesulfonyl)-L-valyl-L-leucinol (1.3 g) was dissolved in dimethyl sulfoxide (20 ml) and dichloromethane (10 ml), and triethylamine (1.9 g) was added. The solution was stirred at room temperature while adding a solution (20 ml) of sulfur trioxide-pyridine complex (2.0 g) in dimethyl sulfoxide, which was followed by stirring for 2 hr. After the completion of the reaction, ethyl acetate was added. The mixture was washed with dilute hydrochloric acid, saturated aqueous solution of sodium hydrogencarbonate and saturated brine, and dried over anhydrous magnesium sulfate. The solvent was evaporated under reduced pressure, and the residue was washed with a mixed solution of hexane-ethyl acetate to give 0.98 g of N-(2-naphthalenesulfonyl)-L-valyl-L-leucinal . as white crystals. [Step 1]

$^1$H-NMR (DMSO-$d_6$ 270MHz) δ: 0.42 (d, 3H, J=6.3Hz), 0.55 (d, 3H, J=6.3Hz), 0.84 (d, 3H, J=6.6Hz), 0.88 (d, 3H, J=6.6Hz), 0.93-1.12 (m, 2H), 1.14-1.28 (m, 1H), 1.82-2.00 (m, 1H), 3.63-3.72 (m, 2H), 7.62-8.40 (m, 9H), 9.02 (s, 1H).

Anal. ($C_{21}H_{28}N_2O_4S$) C, H, N.

In the same manner as in Step 1 except that 4-fluorobenzenesulfonyl chloride was used instead of 2-naphthalenesulfonyl chloride of Step 1, N-(4-fluorophenylsulfonyl)-L-valyl-L-leucinal (Compound 1) was obtained as white crystals. [Step 2]

$^1$H-NMR (DMSO-$d_6$ 270MHz) δ: 0.74 (d, 3H, J=5.9Hz), 0.80 (d, 6H, J=6.4Hz), 0.85 (d, 3H, J=6.8Hz), 1.14-1.46 (m, 3H), 1.81-1.93 (m, 1H), 3.56-3.62 (dd, 1H, J=6.6, 9.5Hz), 3.80-3.88 (m, 1H), 7.33-7.42 (m, 2H), 7.79-7.86 (m, 2H), 7.96 (d, 1H, J=9.8Hz), 8.27 (d, 1H, J=7.3Hz), 9.14 (s, 1H).

Anal. ($C_{17}H_{25}FN_2O_4S$) C, H, N.

Example 2 (Tablet)

| | |
|---|---|
| Compound 1 | 30 mg |
| Lactose | 80 mg mg |
| Starch | 17 |
| Magnesium stearate | 3 mg |

Using the above ingredient as the material for one tablet, tablets are prepared by a conventional method. Where necessary, sugar coating may be applied.

Example 3 (Eye drop)

| Compound 1 | 50 mg |
|---|---|
| Boric acid | 700 mg |
| Sodium tetraborate | q.s. |
| Sodium chloride | 500 mg |
| Hydroxymethylcellulose | 500 mg |
| Disodium edetate | 0.05 mg |
| Benzalkonium chloride | 0.005 mg |
| Sterile purified water | total 100 ml |

The above ingredients are admixed by a conventional method to give a suspension for eye drop.

Ref. Experimental Example 1 Effect of cysteine protease inhibitor on angiogenesis in cornea of guinea pig by transplantation of basic FGF (bFGF)

(Test Method)

(1) Method for preparing pellets for transplantation

A solution (5 μl) of 8% ethylene vinyl acetate copolymer (EVA) in dichloromethane was dropped on a plate made of Tefron, air-dried, and 0.0167 w/v% bFGF (3 μl) was dropped thereon and air-dried. After drying, the obtained product was rounded into a small rod to give a bFGF (500 ng)-containing pellet.
Using a test solution instead of 0.0167 w/v% bFGF, a test solution-containing pellet was prepared in the same manner as above. The test solution-containing pellets were prepared to contain 27 mer calpastatin peptide (0.03 μmole and 0.1 μmole/pellet, Sigma) and leupeptin (0.1 μmole/pellet, PEPTIDE INSTITUTE, INC.).
As a control, a pellet made of EVA alone was prepared and used as a vehicle pellet.

(2) Transplantation of pellet into cornea of guinea pig

The procedure followed the method of M. Kusaka et al. [Biochem. Biophys. Res. Comm., vol. 174, pp. 1070-1076 (1991)]. That is, male guinea pigs weighing 300-400 g were anesthetized with a 1:1 mixture of Ketalar 50 (ketamin hydrochloride, Sankyo Company, Limited) and Celactal (xylazine hydrochloride, Bayer, Ltd.). A pocket was formed in the intercellular layer of corneal stroma layer of both eyes from corneal limbus to the center of cornea, using a 0.5 mm width ophthalmic spatula. A test solution-containing pellet was inserted into the pocked thus formed, and a bFCF-containing pellet was inserted in adjacency thereto. For prevention of infection, ofloxacin eye ointment [Tarivid eye ointment (ofloxacin 0.3%, manufactured by Santen Pharmaceutical Co., Ltd.) was instilled once immediately after insertion of the pellets, Thereafter, 0.3% lomefloxacin (lomeflon ophthalmic otologic solution, manufactured by Senju Pharmaceutical Co., Ltd.) was instilled once a day for 5 days.

(3) Evaluation of the effects of cysteine protease inhibitor

The effect of the cysteine protease inhibitor was observed with a slit lamp. The blood vessel newly formed in cornea which is an avascular tissue had high permeability, and the wet weight and plasma content were expected to elevate due to incurrent of plasma components as a result of high permeability. At 9 days posttransplantation of pellet, guinea pigs were euthanized and cornea was collected. The obtained cornea was weighed (wet weight). Then, it was homogenized, and subjected to centrifugation. The obtained water soluble protein was separated by SDS polyacrylamide gel electrophoresis. After electrophoresis, the gel was stained with Coomassie Brilliant Blue. By image analysis (NIH Image 1.31), albumin which is one of the major proteins in plasma was quantitatively assayed. As the standard substance, guinea pig serum albumin was used.

(Test Results)

(1) Observation of cornea with a slit lamp

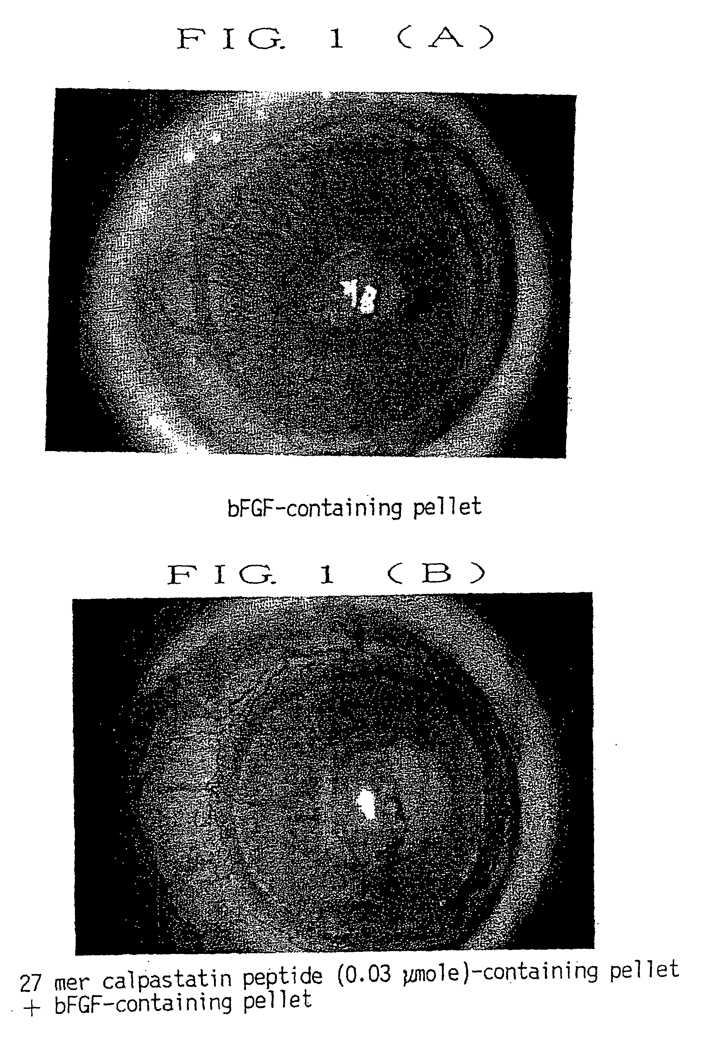

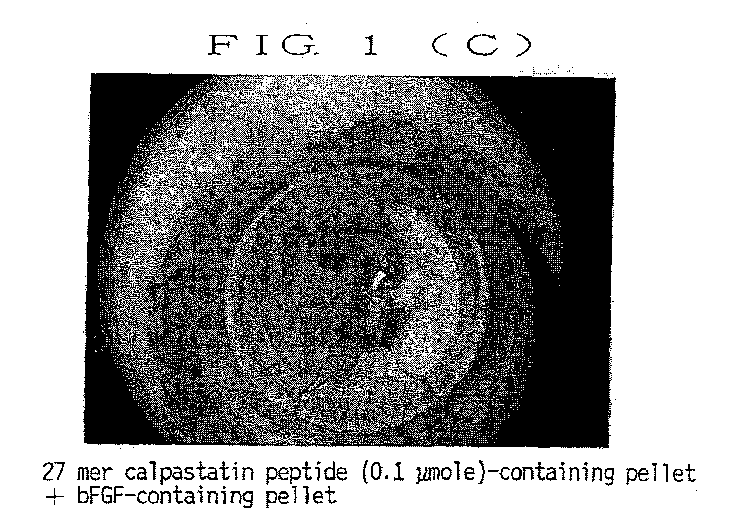

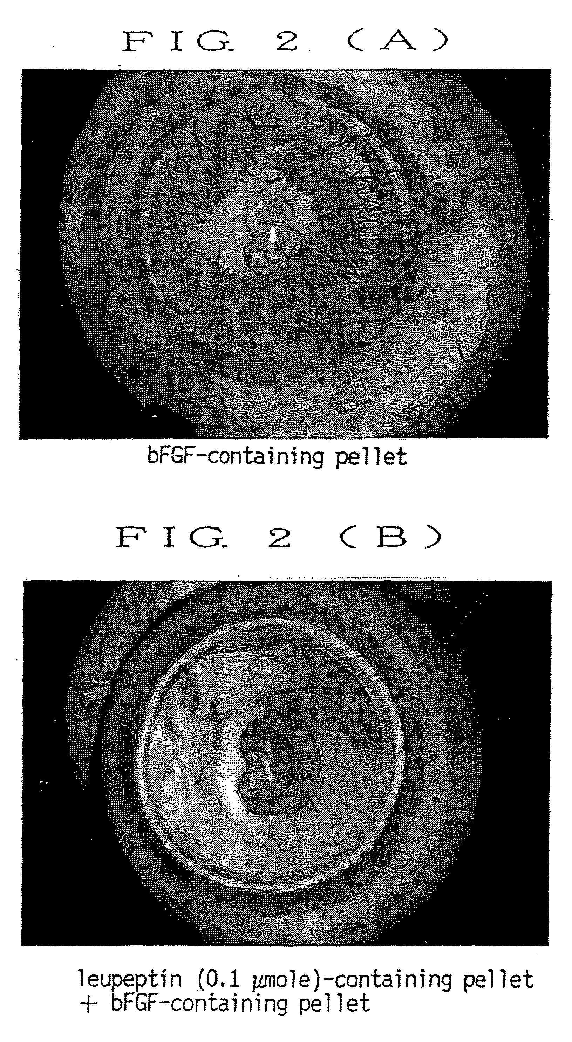

The cornea of vehicle pellet transplantation group showed no changes as compared to the cornea of the untreated group. The bFGF-containing pellet transplantation group (hereinafter sometimes referred to as control group) showed appearance of blood vessel in the neighborhood of bFGF-containing pellet transplantation site from 4 days after transplantation. At 9 days posttransplantation, blood vessels were newly formed radially in the entirety of the cornea of guinea pigs. The group which underwent transplantation of 27 mer calpastatin peptide-containing pellet or leupeptin-containing pellet together with bFGF-containing pellet showed appearance of blood vessel in all groups. Compared to the control group, however, the degree of appearance was mild, and new formation of blood vessels was found mainly in corneal limbus. The corneas at 9 days posttransplantation of guinea pig are shown in Figs. 1 and 2.

(2) Wet weight of cornea

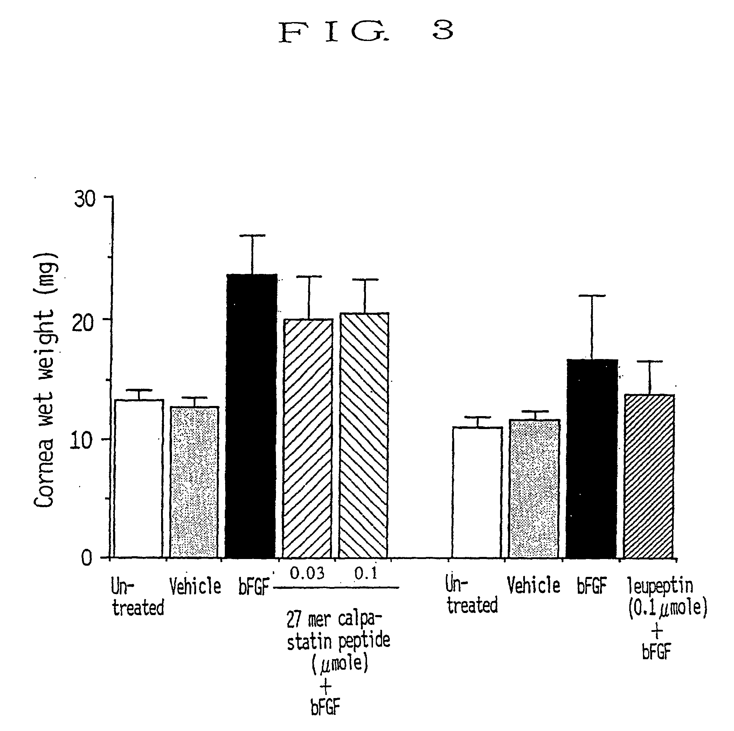

The results are shown in Fig. 3. The wet weight of cornea was almost the same between the untreated group and vehicle pellet transplantation group, and no influence by transplantation of vehicle pellet was found. In contrast, the wet weight of cornea of the control group increased from the weight of the untreated group and vehicle pellet transplantation group. The group which underwent transplantation of 27 mer calpastatin peptide-containing pellet or leupeptin-containing pellet together with bFGF-containing pellet showed suppressed increase in the wet weight of cornea as compared to the control group.
It was clarified therefrom that 27 mer calpastatin peptide and leupeptin suppressed increase in wet weight of cornea caused by angiogenesis.

(3) Amount of albumin in cornea

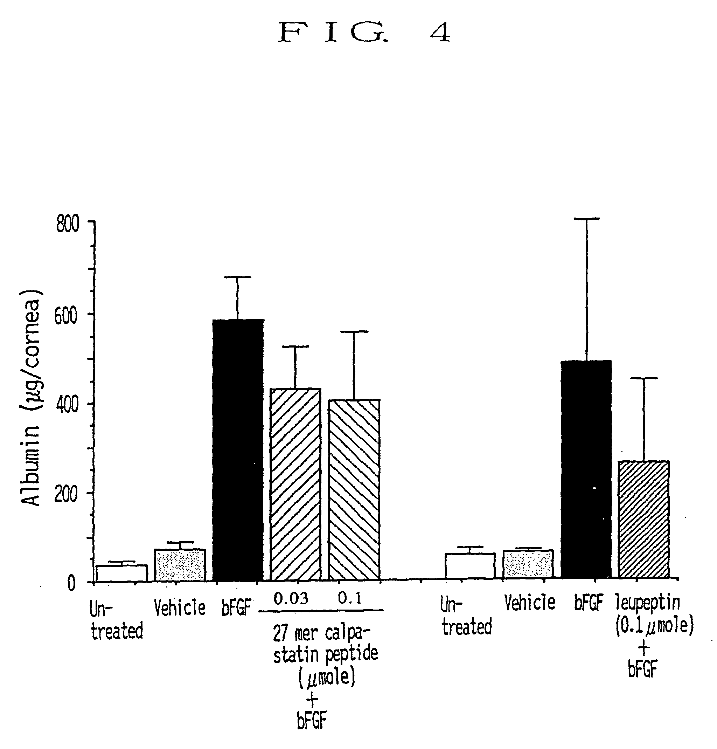

The results are shown in Fig. 4. The amount of albumin of untreated group was very small and was $35.2\pm9.6$ (S.D.) or $56.1\pm13.5$ (S.D.). This was because cornea is an avascular tissue. Meanwhile, that of the group which underwent transplantation of vehicle pellet showed a small increase from the weight of the untreated group. This was supposedly attributable to surgical stimulation during pellet transplantation. In contrast, that of the control group was about 17 or 9 times greater than the untreated group, and about 8 times greater than the vehicle pellet transplantation group. This was because vascular permeability was high in newly formed blood vessels, and albumin which is the main component of plasma leaked. The group transplanted with 27 mer calpastatin peptide-containing pellet and leupeptin-containing pellet together with bFCF-containing pellet showed suppressed increase in the amount of albumin in cornea as compared to the control group.
It was clarified from the above that a cysteine protease inhibitor suppressed angiogenesis.

Experimental Example 1

The biological activity of the compound of the invention is shown in the following. The compound of the invention and salts thereof show thiol protease-inhibitory activity. The inhibitory activity against calpain was determined. The results are shown in Table 1.

μ-Calpain-inhibitory activity

The activity of μ-calpain (Nakalai Tesque) was assayed in accordance with the procedure described in the literature [Anal. Biochem., 208, 387-392 (1993)]. Thus, to a solution containing 0.5 mg/ml casein, 50 mM Tris-HCl (pH 7.4), 20 mM dithiothreitol, and 4 mM calcium chloride was added 2.5 μl of a dimethyl sulfoxide solution containing a varying concentration of the test drug as well as 0.03 unit of μ-calpain to initiate the reaction. The final liquid volume was 250 μl. After 60 minutes of reaction at 30°C, 100 μl of the reaction mixture was transferred to another vessel, to which 50 μl of purified water and 100 μl of 50% Coumassie brilliant blue solution were added. The mixture was allowed to stand at room temperature for 15 minutes and the absorbance was measured at 595 nm. As a control, 2.5 μl of dimethyl sulfoxide not containing the test drug was added and the mixture was treated in the same manner as above. The absorbance value thus found was used as the control value. Similarly, the value found by adding 0.2 mM EDTA in lieu of 4 mM aqueous calcium chloride solution was used as the blank value. The inhibition rate was calculated by means of the following equation and plotted against concentration on log paper and the amount necessary for 50% inhibition ($IC_{50}$) was determined.

$$\text{Inhibition rate (\%)} = (1 - \frac{\text{Measured value - blank value}}{\text{Control value - blank value}}) \times 100$$

Table 1

| Calpain 50% inhibitory concentration ($IC_{50}$) | | | |
|---|---|---|---|
| Test drug | (M) | Test drug | (M) |
| | | Compound 1 | $7.5 \times 10^{-9}$ |

As is evident from the above experimental results, the cysteine protease inhibitory compound to be used in the present invention showed no toxicity to human and animals.

Having inhibitory activity against cysteine proteases such as calpain, the compound of the invention and salts thereof are useful as prophylactic or therapeutic agents for a variety of cysteine protease-associated diseases, such as ischemic diseases, inflammatory diseases, muscular dystrophy, cataract, immune diseases, essential hypertension, Alzheimer's disease, subarachnoid hemorrhage, and osteoporosis, in mammals (e.g. mouse, rat, rabbit, dog, cat, bovine, swine, and human).

The angiogenesis inhibitor of the present invention suppresses new formation of blood vessels in the living tissues, so that it can be used as a superior therapeutic or prophylactic agent of angiogenesis associated with wound healing, inflammation, growth of tumor and the like; and angiogenesis as seen in diabetic retinopathy, prematurity retinopathy, retinal venous occlusion and senile discoid macular degeneration as well as for prevention of metastasis of tumors.

## Claims

1.    N-(4-Fluorophenylsulfonyl)-L-valyl-L-leucinal or a salt thereof.

2.    A pharmaceutical composition comprising N-(4-Fluorophenylsulfonyl)-L-valyl-L-leucinal or a salt thereof.

3.    Use of N-(4-Fluorophenylsulfonyl)-L-valyl-L-leucinal or a salt thereof as an angiogenesis inhibitor.

4.    Use of N-(4-fluorophenylsulfonyl)-L-valyl-L-leucinal or a salt thereof as a calpain inhibitor.

5.    Use of N-(4-fluorophenylsulfonyl)-L-valyl-L-leucinal or a salt thereof as a cysteine protease inhibitor.

## Patentansprüche

1.    N-(4-Fluorphenylsulfonyl)-L-valyl-L-leucinal oder ein Salz davon.

2.    Pharmazeutische Zusammensetzung umfassend N-(4-Fluorphenylsulfonyl)-L-valyl-L-leucinal oder ein Salz davon.

3.    Verwendung von N-(4-Fluorphenylsulfonyl)-L-valyl-L-leucinal oder einem Salz davon als Angiogeneseinhibitor.

4.    Verwendung von N-(4-Fluorphenylsulfonyl)-L-valyl-L-leucinal oder einem Salz davon als Calpaininhibitor.

5.    Verwendung von N-(4-Fluorphenylsulfonyl)-L-valyl-L-leucinal oder einem Salz davon als Cysteinproteaseinhibitor.

## Revendications

1.    N-(4-fluorophénylsulfonyl)-L-valyl-L-leucinal, ou l'un de ses sels.

2.    Composition pharmaceutique comprenant du N-(4-fluorophénylsulfonyl)-L-valyl-L-leucinal, ou l'un de ses sels.

3. Utilisation du N-(4-fluorophénylsulfonyl)-L-valyl-L-leucinal ou d'un de ses sels en tant qu'inhibiteur de l'angiogenèse.

4. Utilisation du N-(4-fluorophénylsulfonyl)-L-valyl-L-leucinal ou d'un de ses sels en tant qu'inhibiteur de la calpaïne.

5. Utilisation du N-(4-fluorophénylsulfonyl)-L-valyl-L-leucinal ou d'un de ses sels en tant qu'inhibiteur de la cystéine-protéase.

# F I G. 1 (A)

bFGF-containing pellet

# F I G. 1 (B)

27 mer calpastatin peptide (0.03 μmole)-containing pellet + bFGF-containing pellet

# F I G. 1 （C）

27 mer calpastatin peptide (0.1 μmole)-containing pellet
+ bFGF-containing pellet

F I G. 2 (A)

bFGF-containing pellet

F I G. 2 (B)

leupeptin (0.1 $\mu$mole)-containing pellet
+ bFGF-containing pellet

F I G. 3

F I G. 4